# FASCICULE DE BREVET EUROPEEN

(11) **EP 0 860 153 B1**
(45) Date de publication et mention de la délivrance du brevet: **28.11.2001**
(21) Numéro de dépôt: 98420026.1
(22) Date de dépôt: 10.02.1998
(51) Int. Cl.: A61F 5/01

(54) **Genouillère avec bandes de dérotation**
Kniegelenksbandage mit Derotationsbändern
Knee brace with derotation bands

(30) Priorité: 13.02.1997 FR 9701911
(43) Date de publication de la demande: 26.08.1998
(73) Titulaire: RICHARD FRERES S.A., 42530 Saint Genest Lerpt (FR)
(72) Inventeur: Richard, Dominique, 42160 Bonson (FR)
(74) Mandataire: Perrier, Jean-Pierre

(56) Documents cités:
- US-A- 4 353 362
- US-A- 5 024 216
- US-A- 5 086 761
- US-A- 5 399 153
- US-A- 5 512 039

## Description

L'invention concerne le domaine des genouillères assurant le traitement orthopédique des entorses, la rééducation postopératoire ou compensant les pathologies méniscales et les laxités chroniques.

De telles genouillères doivent assurer le positionnement de la rotule tant verticalement que transversalement et suppléer aux déficiences ligamentaires de l'articulation fémoro-tibiale, tout en leur laissant la mobilité nécessaire aux mouvements.

On connaît divers types de genouillères qui peuvent se ranger en deux catégories, celles réalisées à partir d'éléments rigides, et celles réalisées à partir d'une nappe souple.

Les genouillères à éléments rigides sont composées de deux éléments, en forme de coque sensiblement semi-cylindriques, qui sont plaqués sur le haut et sur le bas de l'articulation par des sangles. Le serrage des sangles assure également la mise en conformation des coques sur la jambe. Le contact des coques avec la peau n'est pas agréable et l'adaptation à la morphologie n'est pas parfaite de sorte que, pour parvenir à la contention recherchée, il faut serrer fortement les sangles, en amplifiant la sensation de carcan. En pratique, pour obtenir une bonne adaptation, de telles genouillères sont réalisées sur mesure.

Les genouillères réalisées à partir d'une nappe souple en tissu, élastique au moins dans un sens, ont, en général, une forme tubulaire tronconique allant en s'évasant vers le haut et comportent un évidement central pour le passage de la rotule. Cet évidement est, au moins partiellement, entouré par un coussin amortisseur et de recentrage patélaire, et est bordé par deux poches verticales dans lesquelles sont disposées des baleines composées de deux parties, respectivement, supérieure et inférieure, reliées par une articulation, disposée sensiblement au niveau de l'évidement rotulien.

Pour éviter que, lors de la flexion du genou, les articulations des baleines s'écartent vers l'extérieur, en augmentant la mobilité transversale de l'articulation, il est connu de rapporter sur la genouillère une ou deux bandes de dérotation qui, passant sur les articulations des baleines pour les plaquer contre le membre, décrivent un parcours en "S" au cours duquel elles se croisent au dessus ou au dessous de la rotule. Les extrémités de ces bandes sont munies de moyens auto-agrippants permettant leur fixation avec des moyens complémentaires de la genouillère.

Ce type de genouillère est plus agréable à porter, s'adapte mieux aux variations de la morphologie mais présente divers inconvénients. Les premiers dépendent de sa forme tubulaire. En effet celle-ci oblige à introduire la jambe dans la genouillère, c'est-à-dire à procéder à des mouvements qui sont douloureux après une opération ou mal commode avec le port de chaussures, comme c'est le cas pour un sportif. Par ailleurs, ce mode de mise en place empêche toute pose sur la jambe d'un vêtement et par exemple sur une tenue de sport. Un autre inconvénient affecte le confort et résulte de la présence de la nappe derrière le creux poplité qui, lorsque la jambe est en flexion, forme des bourrelets traumatisants.

La deuxième série d'inconvénients est inhérente aux deux bandes de dérotation qui, lors des mouvements de flexion-extension de la jambe et sous l'action des mouvements angulaires des éléments des baleines, sont soumises à des mouvements verticaux tendant à les déplacer et à les éloigner de la zone d'articulation de ces baleines. Pour éviter que ces zones d'articulation s'écartent de la jambe, en diminuant et voire même annulant le maintien du genoux, le porteur doit procéder fréquemment à des réajustements de la position de ces bandes, ce qui s'avère astreignant et lassant.

Enfin, dans certaines genouillères, les bandes de dérotation passant et, éventuellement, se croisant derrière le genou, au niveau du creux poplité, et s'ajoutant parfois à la partie arrière de la nappe tubulaire de la genouillère, forment, lors de la flexion du genou, un amas textile dont l'accumulation dans le creux poplité augmente l'inconfort et génère une source de douleur.

Une telle genouillère est divulguée dans le document US-A-5,399,153.

La présente invention a pour objet de remédier à cela, en fournissant une genouillère s'adaptant aisément à la morphologie, ne nécessitant pas de mouvements du membre pour être mis en place, assurant un maintien constant et ne générant aucune gêne ou inconfort.

Dan la genouillère selon l'invention, la nappe est constituée par un flan plan de forme générale rectangulaire comportant, sur chacun de ses côtés, une échancrure centrale bordée par deux brides transversales de serrage et de fixation, les deux brides disposées d'un même côté étant décalées verticalement par rapport aux brides disposées de l'autre côté, et, sur sa face antérieure, des tronçons de ruban à bouclettes disposés par paire, au dessus et au dessous de l'évidement rotulien, pour coopérer chacun avec un tronçon de ruban auto-agrippant disposé à l'extrémité de la bride correspondante, tandis que chacune des poches pour les baleines comporte, au niveau du centre de l'évidement rotulien, des moyens pour fixer la bande de dérotation croisant sur elle.

La mise en place de cette genouillère s'effectue par enveloppement et ne nécessite donc aucun mouvement du membre, ce qui permet, dans le cas d'un traitement postopératoire, de la mettre en place sans générer une quelconque douleur chez le patient.

Le positionnement et la fixation de chacune de ses extrémités est assuré par deux brides parallèles s'enroulant sur le membre de façon inverse. Avec cet aménagement, la genouillère occupe une position stable sur le membre et n'a pas tendance à pivoter autour de ce membre, puisque les efforts de réaction de chacune des brides d'une paire de brides sont opposés et s'annulent.

Enfin, la fixation des bandes de dérotation au niveau de l'évidement rotulien garantit le positionnement de ces bandes sur les articulations des baleines, et, en conséquence, le maintien procuré par l'orthèse, et permet de donner à la partie de bande passant derrière le genou une trajectoire dégageant le creux poplité. Grâce à ce dégagement et à l'échancrure de la nappe, lors de la flexion du genou, le creux poplité est totalement libéré et le patient n'est soumis à aucune gêne ou inconfort.

En raison de sa structure particulière, cette genouillère peut, par exemple pour la pratique sportive, être mise en place sur un vêtement.

Dans une forme d'exécution de l'invention, les moyens pour fixer chaque bande de dérotation sur une poche à baleine sont constitués par un tronçon de ruban, auto-agrippant sur ses deux faces, aptes à coopérer, par une face, avec les bouclettes d'un tronçon de velours à bouclettes disposé sur la face antérieure de chaque poche et, par l'autre face, avec les bouclettes saillants de la face interne de chaque sangle de dérotation.

Ces tronçons de ruban auto-agrippant forment des pastilles d'accrochage qui s'opposent à tout glissement des sangles de dérotation pendant le port de la genouillère. Ces pastilles permettent, selon la zone précise où elles sont accrochées sur les poches, de modifier la trajectoire des sangles en fonction des besoins. Des pastilles similaires peuvent d'ailleurs coopérer avec les autres tronçons de ruban à bouclettes, soit pour mieux positionner la trajectoire des bandes de dérotation, soit pour donner à ces bandes des trajectoires particulières, soit pour fixer les brides de dérotation.

D'autres caractéristiques et avantages ressortiront de la description qui suit en référence au dessin schématique annexé représentant à titre d'exemple une forme d'exécution de la genouillère selon l'invention.

Figure 1 est une vue de face en élévation avec coupe partielle des différents éléments de la genouillère.

Figure 2 est une vue en perspective de la genouillère lorsqu'elle est mise en place.

Figure 3 est une vue par l'arrière de la genouillère lorsqu'elle est mise en place sur un membre.

Cette genouillère comprend un flan plan 2, des baleines de renfort 3, des bandes de dérotation 4 et des pastilles d'accrochage 5.

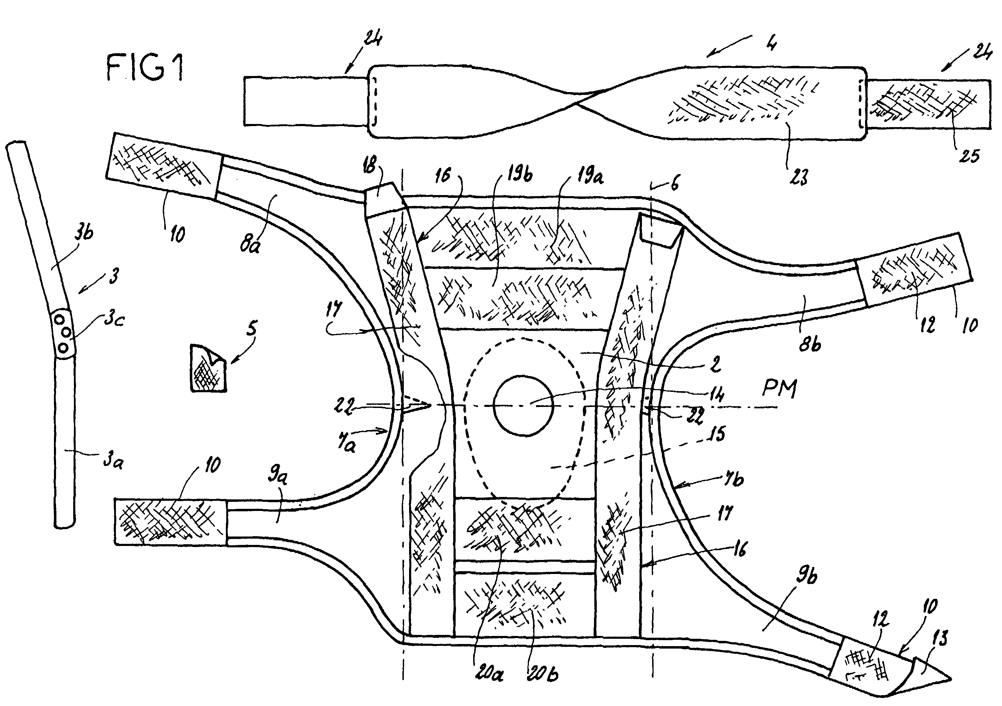

Le flan 2 est réalisé en tissu extensible dans les deux sens et, comme représenté par les traits mixtes 6 à la figure 1, présente une forme générale rectangulaire. Il comporte, sur chacun de ses côtés, une échancrure 7a, 7b bordée par deux brides respectivement 8a, 9a et 8b, 9b. Les deux brides par exemple, celle 8a, 9a disposées d'un côté, sont décalées verticalement par rapport aux brides 8b, 9b disposées de l'autre côté. A son extrémité libre chaque bride est solidaire d'une patte d'accrochage 10 formée, vers l'extérieur, par un ruban auto-agrippant 12 et, vers l'intérieur, par un ruban à bouclettes 13.

Dans sa partie centrale, la nappe 2 comporte un évidement 14 pour le passage de la rotule. Cet évidement est entouré par un coussin amortisseur et annulaire 15, en silicones ou gonflable. Ce coussin est lui-même bordé par deux poches sensiblement verticales 16. La face antérieure de chaque poche 16 est constituée par une nappe de velours à bouclettes 17 qui, dans la forme d'exécution représentée, s'étend sur toute la longueur de la poche. A son extrémité supérieure, chaque poche est munie d'un rabat 18 en velours auto-agrippant. Chaque poche est destinée à recevoir une baleine 3 composée de deux parties 3a, 3b reliée par une articulation 3c, pouvant être double, comme représenté, ou à angulation limitée. Ce type de baleine articulée est bien connue dans l'état de la technique et n'a pas besoin d'être décrit plus en détail.

Sur la face antérieure de la nappe 2 et de part et d'autre de l'évidement 14 sont également rapportées, une paire supérieure 19a, 19b et une paire inférieure 20a, 20b de tronçons transversaux de velours à bouclettes. Le bord de chaque échancrure 7a, 7b comporte sensiblement au niveau du centre de l'évidement rotulien 14, une pince 22 dont l'utilité sera précisée plus loin. Dans la forme d'exécution représentée cette pince est réalisée en pratiquant une découpe en "V" dans la nappe et en cousant les bords de cette découpe après les avoir superposés.

Chacune des sangles de dérotation 4 est constituée par un ruban élastique comportant, sur sa face destinée à venir en contact avec la genouillère, des bouclettes 23. A chacune de ses extrémités, la sangle est munie d'une patte d'accrochage 24 formée par un tronçon de velours à crochets dont les crochets 25 sont tournés du même côté que les bouclettes 23 de la sangle.

Enfin chaque pastille d'accrochage 5 est formée par un tronçon de ruban auto-agrippant double face, c'est-à-dire comportant des crochets saillants de chacune de ses faces.

Lorsque la nappe est mise en place sur le genou, sa fixation est assurée par l'accrochage des pattes 10 de ses brides 8a, 8b et 9a, 9b sur, respectivement, les tronçons 19a, 19b, et 20a, 20b de velours à bouclettes.

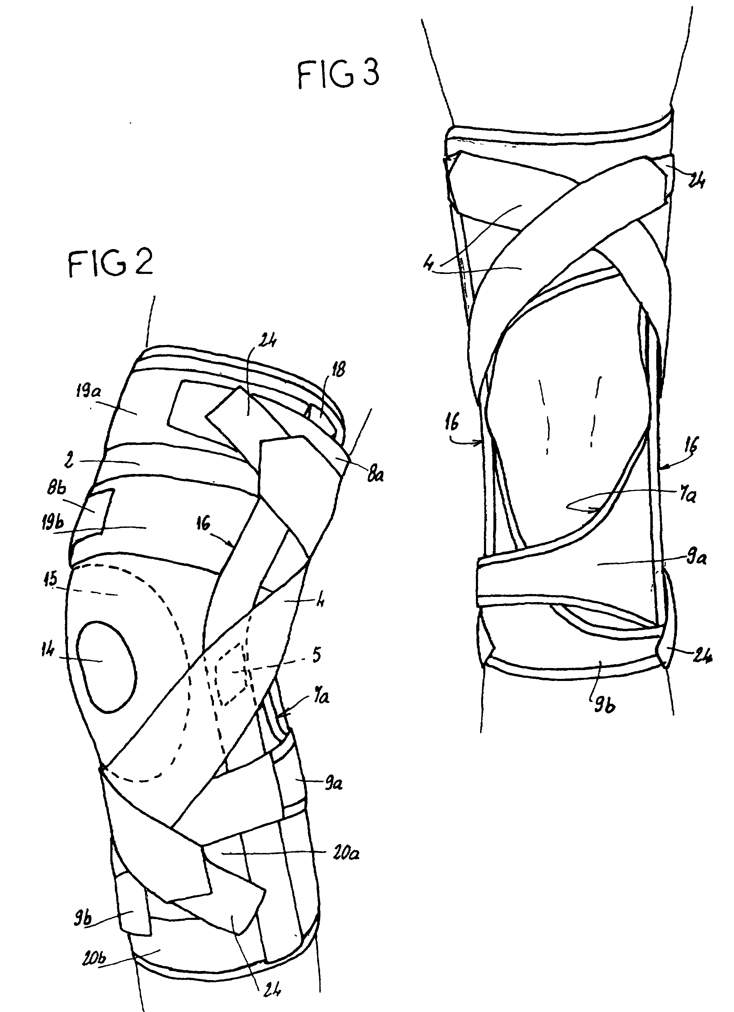

La figure 2 montre bien que dans ces conditions les échancrures 7a, 7b dégagent totalement la partie arrière du genou. Il est ici précisé que grâce aux plis 22 formés dans la nappe 2, lorsque celle-ci est en position d'utilisation, les bords gansés des échancrures 7a, 7b ne tendent pas à s'insérer entre la genouillère et la peau mais, au contraire, tendent à s'écarter de la peau pour éviter de former une surépaisseur pouvant blesser le porteur.

A ce stade de la mise en place de la genouillère, il est procédé au positionnement des pastilles d'accrochage 5 sur la face antérieure en bouclettes 17 des poches 16. Chaque pastille est disposée, verticalement, de manière à être sensiblement dans le plan médian transversal PM de la genouillère passant par le centre de l'évidement rotulien 14 et, transversalement, en fonction de la trajectoire que l'on veut donner à la bande de dérotation correspondante.

Dans le cas d'un maintien sous-rotulien, comme montré à la figure 2, chacune des bandes de dérotation est d'abord accrochée par sa patte inférieure 24 sur l'un des tronçons inférieurs 20a, 20b de velours à bouclettes, puis est passée en diagonale au dessous la rotule, de manière à croiser la poche 16 correspondante. Elle est passée derrière la cuisse de manière à venir s'accrocher, par son autre patte 24, sur l'un ou l'autre des tronçons 19a, 19b ou sur les bouclettes 17 d'une poche 16. A son croisement avec la poche 16 chaque bande 4 est liée à cette poche par la pastille auto-accrochante 5. Grâce à cela, la bande est parfaitement calée en position et ne risque pas de glisser vers le bas. Elle assure donc parfaitement sa fonction de placage de l'articulation des baleines contre le membre.

La fixation de la bande à cet endroit permet de régler sa trajectoire supérieure de manière qu'elle vienne, comme montré à la figure 3, en bordure de l'échancrure 7a ou 7b correspondante, en dégageant ainsi totalement le creux poplité.

Cette possibilité de réglage est particulièrement intéressante dans le cas d'un maintien sus-rotulien, dans lequel les bandes 4 se croisent sur l'avant, au dessus du genou, et sur l'arrière, au dessous du creux poplité, car cela permet de dégager ce creux poplité en supprimant toute gêne.

Les pastilles d'accrochage 5 peuvent, en cas de besoin, être disposées sur l'un ou l'autre des tronçons 19a, 19b, 20a, 20b pour modifier la trajectoire des sangles de dérotation et, voir même, celle des brides de fixation.

Dans une variante de réalisation, chaque sangle de dérotation est fixée par des coutures à la poche 16 sous-jacentes. Comme dans la forme d'exécution représentée cette fixation est assurée sensiblement au niveau du centre de l'évidement rotulien, c'est-à-dire au niveau de l'articulation des deux parties des baleines 3.

En raison de sa structure, cette genouillère peut être utilisée pour la rééducation post-chirurgicale, pour le traitement orthopédique des entorses, pour le traitement des laxités chroniques, pour la reprise du sport et pour le traitement de pathologies méniscales. Pour la pratique du sport, elle peut être mise en place immédiatement avant l'effort et voir même sur un vêtement, puisque, grâce à sa structure, elle ne risque pas de créer une gêne et un inconfort en plaquant contre la peau la partie de vêtement juxtaposée au creux poplité.

## Revendications

1. Genouillère avec bandes de dérotation composée, d'une part, d'une nappe (2) en tissu extensible comprenant un évidement rotulien (14) avec un coussin amortisseur (15) et bordé par deux poches (14), sensiblement verticales et aptes chacune à recevoir une baleine (3) en deux parties, ces deux parties (3a, 3b) étant liées par une articulation (3c) disposée sensiblement au niveau du plan médian horizontal de l'évidement rotulien (14), et, d'autre part, d'au moins deux bandes (4) de dérotation dont les extrémités sont munies de rubans auto-agrippants (25) aptes à coopérer avec des tronçons de velours à bouclettes fixés sur la nappe, la nappe (2) étant constituée par un flan plan de forme générale rectangulaire comportant, sur chacun de ses côtés, une échancrure centrale (7a, 7b) bordée par deux brides transversales (8a, 8b et 9a, 9b) de serrage et de fixation, les deux brides (8a, 8b) disposées d'un même côté étant décalées verticalement par rapport aux brides (9a, 9b) disposées de l'autre côté, et, sur sa face antérieure, des tronçons de ruban à bouclettes disposés par paire (19a, 19b et 20a, 20b), au dessus et au dessous de l'évidement rotulien (14), pour coopérer chacun avec un tronçon (12) de ruban auto-agrippant disposé à l'extrémité de la bride correspondante et chacune des poches (16) pour les baleines comportent, au niveau du centre de l'évidement rotulien, des moyens pour fixer la bande de dérotation (4) croisant sur elle.

2. Genouillère avec bandes de dérotation selon la revendication 1, **caractérisée en ce que** les moyens pour fixer chaque bande (4) de dérotation sur une poche (16) à baleine sont constitués par un tronçon (5) de ruban auto-agrippant sur ses deux faces apte à coopérer, par une face, avec les bouclettes d'un tronçon (17) de velours à bouclettes disposé sur la face antérieure de chaque poche (16) et, par l'autre face, avec les bouclettes (23) saillants de la face interne de chaque sangle de dérotation (4).

3. Genouillère avec bandes de dérotation selon la revendication 1 **caractérisée en ce que** les moyens pour fixer chaque bande (4) de dérotation sur une poche (16) à baleine sont constitués par des coutures.

4. Genouillère avec bandes de dérotation selon la revendication 1, **caractérisée en ce que** le bord de chaque échancrure (7a, 7b) comporte une pince (22) disposée sensiblement au niveau du centre de l'évidement rotulien.

## Patentansprüche

1. Knieschiene mit Derotationsbändern, gebildet einerseits aus einem Tuch (2) aus dehnbarem Gewebe, umfassend eine Kniescheibenaussparung (14) mit einem Dämpfungskissen (15), das von zwei Taschen (16) begrenzt ist, welche im Wesentlichen vertikal und dazu ausgelegt sind, jeweils ein Stäbchen (3) aus zwei Teilen aufzunehmen, wobei diese zwei Teile (3a, 3b) durch ein Gelenk (3c) verbunden sind, das im Wesentlichen im Bereich der horizontalen Mittelebene der Kniescheibenaussparung (14) angeordnet ist, und andererseits aus wenigstens zwei Derotationsbändern (4), deren Enden mit selbstbefestigenden Bändern (25) ausgestattet sind, die dazu ausgelegt sind, mit auf dem Tuch befestigten Schlingensamtabschnitten zusammenzuwirken, wobei das Tuch (2) durch einen flachen Stoff mit allgemeiner Rechteckgestalt gebildet ist, der an jeder seiner Seiten einen zentralen Ausschnitt (7a, 7b) aufweist, welcher von zwei Spann- und Befestigungsquergurten (8a, 8b und 9a, 9b) begrenzt ist, wobei die zwei auf ein und derselben Seite angeordneten Gurte (8a, 8b) vertikal bezüglich der auf der anderen Seite angeordneten Gurte (9a, 9b) versetzt sind, und ferner umfassend an seiner Vorderfläche paarweise angeordnete Schlingenbandabschnitte (19a, 19b und 20a, 20b) über und unter der Kniescheibenaussparung (14) zum jeweiligen Zusammenwirken mit einem am Ende des zugeordneten Gurtes angeordneten Abschnitt (12) selbstbefestigenden Bandes, und wobei jede der Taschen (16) für die Stäbchen im Bereich der Mitte der Kniescheibenaussparung Mittel zur Befestigung des über sie verlaufenden Derotationsbandes (4) umfasst.

2. Knieschiene mit Derotationsbändern nach Anspruch 1,
**dadurch gekennzeichnet, dass** die Mittel zur Befestigung eines jeweiligen Derotationsbandes (4) an einer Stäbchentasche (16) gebildet sind durch einen Abschnitt (5) eines beidseitig selbstbefestigenden Bandes, das dazu ausgelegt ist, an einer Seite mit den Schlingen eines an der Vorderseite jeder Tasche (16) angeordneten Schlingensamtabschnittes (17) zusammenzuwirken und an der anderen Seite mit den an der Innenseite jedes Derotationsriemens (4) vorstehenden Schlingen (23).

3. Knieschiene mit Derotationsbändern nach Anspruch 1,
**dadurch gekennzeichnet, dass** die Mittel zur Befestigung eines jeweiligen Derotationsbandes (4) an einer Stäbchentasche (16) durch Nähte gebildet sind.

4. Knieschiene mit Derotationsbändern nach Anspruch 1,
**dadurch gekennzeichnet, dass** der Rand jedes Ausschnittes (7a, 7b) eine Klemme (22) aufweist, die im Wesentlichen im Bereich der Mitte der Kniescheibenaussparung angeordnet ist.

## Claims

1. A knee brace with derotation bands, composed on the one hand of a sheet (2) of stretchable material having a patellar opening (14) with a shock-absorbent pad (15) and bordered by two pouches (14), substantially vertical and each capable of accommodating a stiffening member (3) in two parts, these two parts (3a, 3b) being connected by a hinge (3c) arranged substantially at the level of the median horizontal plane of the patellar opening (14), and on the other hand of at least two derotation bands (4) the ends of which are provided with self-gripping tapes (25) capable of co-operating with lengths of uncut velvet attached to the sheet, the sheet (2) consisting of a plane matrix of generally rectangular shape comprising, on each of its sides, a central indentation (7a, 7b) bordered by two transverse straps (8a, 8b and 9a, 9b) for tightening and attaching, the two straps (8a, 8b) arranged on the same side being vertically offset relative to the straps (9a, 9b) arranged on the other side and, on its anterior surface, lengths of looped tape arranged in pairs (19a, 19b and 20a, 20b), on top of and beneath the patellar opening (14), for co-operating each with a length (12) of self-gripping tape arranged at the end of the corresponding strap, and each of the pouches (16) for the stiffening members having, at the level of the centre of the patellar opening, means for attaching the derotation bands (4) crossing over it.

2. A knee brace with derotation bands according to Claim 1, **characterised in that** the means for attaching each derotation band (4) to a stiffened pouch (16) comprise a length (5) of self-gripping tape on their two surfaces which is capable of co-operating, via one surface, with the loops of a length (17) of uncut velvet arranged on the anterior surface of each pouch (16) and, via the other surface, with the loops (23) projecting from the inner surface of each derotation band (4).

3. A knee brace with derotation bands according to Claim 1, **characterised in that** the means for attaching each derotation band (4) to a stiffened pouch (16) consist of stitching.

4. A knee brace with derotation bands according to Claim 1, **characterised in that** the edge of each indentation (7a, 7b) comprises a dart (22) arranged substantially at the level of the centre of the patellar opening.
